# EUROPEAN PATENT APPLICATION

(11) **EP 3 756 647 A1**
(43) Date of publication of application: **30.12.2020**
(21) Application number: 18906967.7
(22) Date of filing: 31.10.2018
(51) Int. Cl.: A61K 8/9794, A23L 33/105, A61K 36/888, A61Q 19/08, A61P 17/00

(54) **COMPOSITION CONTAINING SKUNK CABBAGE EXTRACT OR FRACTION THEREOF AS ACTIVE INGREDIENT FOR WRINKLE RELIEF**

(30) Priority: 23.02.2018 KR 20180022051
(71) Applicant: LG Household & Health Care Ltd., Seoul 03184 (KR)
(72) Inventor: CHANG, Yun Hee, Daejeon 34114 (KR); JIN, Mu Hyun, Daejeon 34114 (KR); KIM, Taeyoon, Daejeon 34114 (KR); KIM, Jongwook, Daejeon 34114 (KR); LEE, Jeongho, Daejeon 34114 (KR)
(74) Representative: Goddar, Heinz J.
(86) International application number: PCT/KR2018/013073
(87) International publication number: WO 2019/164090

(57) **Abstract**

The present invention relates to a composition for preventing or improving wrinkles, which comprises a *Symplocarpus foetidus* extract or a fraction thereof as an active ingredient. Specifically, the present invention relates to a cosmetic composition, a food composition, a pharmaceutical composition, and a quasi-drug composition, which comprise a *Symplocarpus foetidus* extract or a fraction thereof as an active ingredient; and to a method for improving wrinkles, comprising administering the composition comprising a *Symplocarpus foetidus* extract or a fraction thereof to a subject excluding humans.

## Description

### [Technical Field]

The present invention relates to a composition for preventing or improving wrinkles, wherein the composition contains a *Symplocarpus foetidus* extract or a fraction thereof as an active ingredient. Specifically, the present invention relates to a cosmetic composition, a food composition, a pharmaceutical composition, and a quasi-drug composition, which contain a *Symplocarpus foetidus* extract or a fraction thereof as an active ingredient; and a method for improving wrinkles, which includes administering the composition containing a *Symplocarpus foetidus* extract or a fraction thereof to a subject excluding humans.

### [Background Art]

Wrinkles can largely be divided into facial expression wrinkles, caused by facial muscles used to make facial expressions, and fine wrinkles that occur locally or over the entire face. Muscles that cause facial expression wrinkles contract and relax by the action of acetylcholine, which is a neurotransmitter secreted from the nerve endings of the neuromuscular junction. Botulinum neurotoxin is the most potent material which is currently used for improving facial expression wrinkles. It cleaves SNAP-25 in the soluble *N*-ethylmaleimide-sensitive factor attachment protein receptor (SNARE; SNAP receptor), which is a protein complex regulating the release of neurotransmitters, and blocks the formation of a SNARE complex by inducing membrane fusion between neuronal synaptic vesicles and presynaptic cells, and consequently prevents the release of acetylcholine to thereby paralyze muscle cells. However, considering that the mechanism of action of botulinum neurotoxin is the cleavage of the irreversible SNARE complex, botulinum neurotoxin is a toxic material, *etc.,* safety issues have been raised continuously. As a result, synthetic peptides (*e.g*., Argireline®) involved in the mechanism of action of botulinum neurotoxin have been developed (Blanes-Mira, etc., International Journal of Cosmetic Science. 24, 303-310, 2002). However, Argireline® has not been satisfactory with its effect on price and has a disadvantage of being a synthetic material.

Unlike facial expression wrinkles, fine wrinkles are known to be caused by a decrease in the amount of collagen, which is a major component of the extracellular matrix. Collagen is a major matrix protein produced by skin fibroblasts, and it accounts for about 30% of the total weight of the proteins in the body. The main functions of collagen are known as mechanical firmness of the skin, resistance of connective tissue and binding forces of tissue, support of cell adhesion, induction of cell division and differentiation (during the growth of organisms or wound healing). Additionally, collagen is a substrate protein(matrix) having an important role in the retention of moisture in the dermal layer, and it is known that these substrate proteins adsorb moisture, and increase the moisture retention capacity inside the structure that they establish, so that the skin can maintain a state containing proper moisture to be involved in maintaining skin elasticity. It is known that the collagen decreases with aging and with photoaging due to ultraviolet irradiation, and the decrease of collagen is closely related to the formation of wrinkles in the skin. In order to solve these problems, materials that promote collagen synthesis have been of great interest. Examples of known conventional materials that promote collagen synthesis include vitamin C, retinoic acid, animal placenta-derived proteins, betulinic acid, chlorella extract, *etc.* However, these materials have limited usage due to safety problems (*e.g.,* irritation, redness, *etc.* during skin application) or the effects are insignificant, and thus the effect of improving skin wrinkles could not substantially be expected. Therefore, there is an urgent need for the development of a novel composition for preventing and improving wrinkles with improved safety *in vivo* and higher effects compared to the existing composition for external skin.
(Patent Document 1) Korean Patent Application Publication No. 2015-0143375
(Patent Document 2) Korean Patent Application Publication No. 2015-0009266

### [Disclosure]

### [Technical Problem]

The present inventors have performed extensive research and development work to discover materials capable of exhibiting an excellent skin-improving effect from natural resources existing in nature. As a result, they have confirmed that a *Symplocarpus foetidus* extract has the effects of reducing muscle contraction and increasing the total amount of collagen biosynthesis, thereby completing the present invention.

### [Technical Solution]

A main object of the present invention is to provide a cosmetic composition for preventing or improving wrinkles, which contains a *Symplocarpus foetidus* extract or a fraction thereof as an active ingredient.

Another object of the present invention is to provide a functional food composition for preventing or improving wrinkles, which contains a *Symplocarpus foetidus* extract or a fraction thereof as an active ingredient.

Still another object of the present invention is to provide a pharmaceutical composition for preventing or improving wrinkles, which contains a *Symplocarpus foetidus* extract or a fraction thereof as an active ingredient.

Still another object of the present invention is to provide a quasi-drug composition for preventing or improving wrinkles, which contains a *Symplocarpus foetidus* extract or a fraction thereof as an active ingredient.

Still another object of the present invention is to provide a method for improving wrinkles, which includes administering the above composition containing a *Symplocarpus foetidus* extract or a fraction thereof to a subject excluding humans.

### [Advantageous Effects]

The *Symplocarpus foetidus* extract according to the present invention has excellent effects of reducing muscle contraction and increasing the total amount of collagen biosynthesis, and thus can be used for cosmetics, pharmaceutical drugs, quasi-drugs, *etc.* for improving skin wrinkles.

### [Brief Description of Drawings]

FIG. 1 shows a graph illustrating the effect of a *Symplocarpus foetidus* extract on the inhibition of muscle contraction according to its concentration.
FIG. 2 shows a graph illustrating the measurement results of the total amount of collagen biosynthesis according to the concentration of a *Symplocarpus foetidus* extract.
FIG. 3 shows images illustrating the visual evaluation of forehead wrinkles using a DSLR after using an essence containing 2% of a *Symplocarpus foetidus* extract.

### [Best Mode for Carrying Out the Invention]

The present inventors have performed extensive research and development work to discover materials capable of exhibiting an excellent skin-improving effect from natural resources existing in nature. As a result, they have confirmed that a *Symplocarpus foetidus* extract has the effects of reducing muscle contraction and increasing the total amount of collagen biosynthesis. From these results, it was confirmed that a *Symplocarpus foetidus* extract can be used as a cosmetic composition for improving wrinkles. The great significance of the present invention lies in that the cosmetic composition containing a *Symplocarpus foetidus* extract has not been known until now and was first discovered by the present inventors.

To achieve the above objects, an aspect of the present invention provides a cosmetic composition for preventing or improving wrinkles, which contains a *Symplocarpus foetidus* extract or a fraction thereof as an active ingredient.

As used herein, the term *"Symplocarpus foetidus"* refers to a perennial monocot plant in the order Alismatales, family Araceae distributed in North America, eastern part of Siberia, and East Asia, and it grows in the shade of mountains throughout the nation of Korea, excluding Jeju Island. It has no stems, and flowers bloom before leaves between February and April. Small flowers appear in the spathe, which is an oval flower cover, which looks like a pocket with red spots. Fruits are berries and ripen to become red in summer.

Although it has been known that the *Symplocarpus foetidus* has mainly sedative and antipyretic effects, the wrinkle improvement effect of *Symplocarpus foetidus* was first identified by the present inventors. For the purpose of the present invention, *Symplocarpus foetidus* is not limited to its kind, but all kinds of *Symplocarpus foetidus* can be used regardless of its source, whether it is one that is cultivated, one that is commercially available, *etc.*

As used herein, the term "extract" refers to a resulting product, such as a liquid component which is obtained by immersing a desired material in various solvents followed by extracting for a predetermined period of time at room temperature, at low temperature, or in a heated state; a solid component which is obtained by removing the solvent from the liquid component; *etc.* Moreover, the extract may be comprehensively interpreted to include all of a diluted solution of the resulting products, a concentrated solution thereof, a crude product thereof, a purified product thereof, *etc.,* in addition to the above resulting products.

In the present invention, the extract refers to a *Symplocarpus foetidus* extract. The *Symplocarpus foetidus* extract can be extracted from various organs (*e.g.,* roots, above-ground parts, stems, leaves, flowers, trunks of fruits, fruit peels, as well as plant tissues) of natural, hybrid, and varietal plants. The *Symplocarpus foetidus* extract can be obtained by extraction with water, various organic solvents, *etc.* In particular, the organic solvent to be used is not particularly limited as long as an extract can be obtained using the solvent, and specifically, it may be water, a polar or non-polar solvent, more specifically, it may be water, a lower alcohol having 1 to 6 carbon atoms (methanol, ethanol, propanol, butanol, *etc*.), or a mixed solvent thereof, and most specifically, it may be methanol or a mixed solvent thereof.

In addition, the method for obtaining the extract is not particularly limited as long as a *Symplocarpus foetidus* extract can be obtained by the method, and specifically, a cold-water precipitation method in which roots, stems, leaves, fruits, flowers, dried products thereof, processed products thereof, *etc.* of the *Symplocarpus foetidus* are immersed in any of the above solvents followed by extraction at room temperature; a heat extraction method; an ultrasonic extraction method performed by applying ultrasonic waves; a reflux extraction method performed using a reflux condenser; *etc.*

As used herein, the term "fraction" refers to a resulting product obtained by performing fractionation to separate a particular component or group of specific components from a mixture containing various components.

In the present invention, the fractionation method for obtaining a fraction is not particularly limited as long as the fraction can exhibit an effect of improving skin wrinkles, and may be performed according to a method commonly used in the art. For example, the fractionation method may be a solvent fractionation method performed by treating various solvents, an ultrafiltration fractionation method performed by passing through an ultrafiltration membrane having a constant molecular weight cut-off value, a chromatography fractionation method performing various kinds of chromatography (prepared for separation according to size, charge, hydrophobicity, or affinity), a combination thereof, *etc.*

In the present invention, the kind of solvent used to obtain the fraction is not particularly limited, and any solvent known in the art may be used. Non-limiting examples of the fraction solvent may include a polar solvent (*e.g.,* water, distilled water, alcohol, *etc*.; a non-polar solvent (*e.g.,* hexane, ethyl acetate, chloroform, dichloromethane, *etc*.); *etc.* These solvents may be used alone or in a combination of two or more kinds. When alcohol is used as a solvent among the solvents for fractionation, a C₁₋₄ alcohol may preferably be used.

The cosmetic composition of the present invention may contain the above extract or a fraction thereof in an amount of 0.0001 wt% to 10 wt%, and specifically 0.0005 wt% to 5 wt%, relative to the total weight of the cosmetic composition.

When the amount is less than 0.0001 wt%, it is undesirable because the desired activity cannot be sufficiently achieved, whereas when the amount exceeds 10 wt%, a distinct increase in the effect as much as the increased content is not expected, and thus may not be economical.

In the present invention, the composition may be characterized in that it improves skin wrinkles.

As used herein, the term "wrinkles" can largely be divided into facial expression wrinkles, caused by facial muscles used to make facial expressions, and fine wrinkles that occur locally or over the entire face due to skin degeneration. Specifically, wrinkles can occur due to genetic causes, a decrease of collagen present in the dermis of the skin, external environment, *etc.* For the purpose of the present invention, the wrinkles may be used in a sense where both facial expression wrinkles and fine wrinkles are included, but are not limited thereto.

In the present invention, the composition may be characterized in that it reduces muscle contraction, thereby reducing facial expression wrinkles.

In an embodiment of the present invention in which a *Symplocarpus foetidus* extract was treated at various concentrations, it was confirmed that muscle contraction was reduced in a concentration-dependent manner.

From the above results, it was confirmed that the *Symplocarpus foetidus* extract can improve facial expression wrinkles.

Additionally, in the present invention, the composition may be characterized in that it promotes collagen synthesis, thereby increasing the total amount of collagen.

In an embodiment of the present invention, it was confirmed that the *Symplocarpus foetidus* extract has an effect of promoting collagen synthesis, thereby increasing the total amount of collagen.

From the above results, it was confirmed that the *Symplocarpus foetidus* extract can improve fine wrinkles.

As used herein, the term "improvement" refers to alleviation of the symptoms of wrinkles on the skin by using the composition of the present invention, and "prevention" includes both preventing the formation of wrinkles and delaying the formation of wrinkles.

The cosmetic composition according to the present invention may be prepared in a formulation selected from the group consisting of a solution, an ointment for external use, cream, foam, a toner, a lotion, a pack, softening water, an emulsion, a makeup base, an essence, a soap, a liquid cleaner, a bath product, sunscreen cream, sun oil, a suspension, an emulsion, a paste, a gel, a lotion, powder, a soap, a surfactant-containing cleanser, oil, powder foundation, emulsion foundation, wax foundation, a patch, and a spray, but the formulation is not limited thereto.

Additionally, the cosmetic composition according to the present invention may further contain at least one cosmetically acceptable carrier that is blended in general skin cosmetics, and as a typical component, for example, oil, water, a surfactant, a moisturizer, a lower alcohol, a thickener, a chelating agent, a pigment, a preservative, a fragrance, *etc.* may be appropriately blended, but the cosmetically acceptable carrier is not limited thereto.

The cosmetically acceptable carrier to be contained in the cosmetic composition of the present invention varies depending on the formulation.

When the formulation of the present invention is an ointment, paste, cream, or gel, as a carrier component, an animal oil, a vegetable oil, wax, paraffin, starch, tragacanth, a cellulose derivative, polyethylene glycol, silicone, bentonite, silica, talc, zinc oxide, or a mixture thereof may be used.

When the formulation of the present invention is powder or a spray, as a carrier component, lactose, talc, silica, aluminum hydroxide, calcium silicate, polyamide powder, or a mixture thereof may be used. In particular, when the formulation of the present invention is a spray, as a carrier component, a propellant such as a chlorofluorohydrocarbon, propane/butane, and dimethyl ether may be additionally contained.

When the formulation of the present invention is a solution or emulsion, as a carrier component, a solvent, a solubilizer, or an emulsifier may be used. For example, water, ethanol, isopropanol, ethyl carbonate, ethyl acetate, benzyl alcohol, benzyl benzoate, propylene glycol, and 1,3-butylglycol oil may be used, and in particular, cottonseed oil, peanut oil, corn seed oil, olive oil, castor oil and sesame oil, glycerol aliphatic ester, polyethylene glycol or fatty acid ester of sorbitan may be used.

When the formulation of the present invention is a suspension, as a carrier component, a liquid diluent (*e.g.,* water, ethanol, and propylene glycol), a suspending agent (*e.g.,* ethoxylated isostearyl alcohol, polyoxyethylene sorbitol ester, and polyoxyethylene sorbitan ester), microcrystalline cellulose, aluminum metahydroxide, bentonite, agar, tragacanth, *etc.* may be used.

When the formulation of the present invention is a soap, as a carrier component, an alkali metal salt of fatty acid, fatty acid hemiester salt, fatty acid protein hydrolyzate, isethionate, lanolin derivative, aliphatic alcohol, vegetable oil, glycerol, sugar, *etc.* may be used.

The cosmetic composition of the present invention may contain, in the *Symplocarpus foetidus* extract or a fraction thereof, a commonly used auxiliary agent, such as a hydrophilic or lipophilic gelling agent, hydrophilic or lipophilic active agent, preservative, antioxidant, solvent, fragrance, filler, blocker, pigment, odorant, dye, *etc.*

To achieve the above objects, another aspect of the present invention provides a functional food composition for preventing or improving wrinkles, which contains a *Symplocarpus foetidus* extract or a fraction thereof as an active ingredient.

As used herein, the terms *"Symplocarpus foetidus",* "extract", "fraction", "wrinkles", "prevention", and "improvement" are as described above.

In the present invention, the functional food composition may have effects such as improving skin wrinkles, *etc.,* but the effects of the functional food composition are not limited thereto.

The functional food composition of the present invention, which contains a *Symplocarpus foetidus* extract or a fraction thereof as an active ingredient, may be contained in the form of an extract thereof, a fraction thereof, or a processed product thereof. Additionally, the composition may contain a sitologically acceptable auxiliary food additive in addition to the active ingredient.

As used herein, the term "auxiliary food additive" refers to an ingredient that can be auxiliarily added to a food. It is added in preparation of each formulation of health functional foods, and one of ordinary skill in the art can appropriately select and use it. Examples of the auxiliary food additive include various nutritional supplements, vitamins, minerals (electrolytes), flavors (*e.g.,* synthetic flavors, natural flavors, *etc.*), coloring agents, filling agents, pectic acid and a salt thereof, alginic acid and a salt thereof, organic acids, protective colloid thickening agents, pH adjusters, stabilizers, preservatives, glycerin, alcohol, carbonating agents used in carbonated beverages, *etc.,* but the types of the auxiliary food additive are not limited by the examples.

The functional food composition of the present invention may include a health functional food. As used herein, the term "health functional food" refers to a food prepared or processed in the form of a tablet, capsule, powder, granule, liquid, pill, *etc.* using raw materials or ingredients that have functionalities useful for the human body. As used herein, the term "functionality" refers to adjusting nutrients for structures and functions of the human body or obtaining useful effects for hygienic use (*e.g*., physiological action). The health functional food of the present invention may be prepared by a method conventionally used in the art, and during the preparation, raw materials and ingredients conventionally added in the art may be added for preparation. Additionally, the types of formulations of the health functional food may be prepared without limitation as long as it can be acknowledged as a health functional food. The food composition of the present invention may be prepared in various types of formulations. Additionally, unlike conventional drugs, the food composition of the present invention has advantages in that there are no side effects, *etc.* that may be caused by long-term administration because the food composition uses food as a raw material. Further, the health functional food of the present invention has excellent portability, and thus it can be consumed as a supplement for the effect of improving skin wrinkles.

There are no limitations on the form that the health functional food of the present invention can have. The health functional food may contain all foods in a conventional sense, and it can be used interchangeably with other terms known in the art *(e.g.,* functional food, *etc.).* In addition, the health functional food of the present invention may be prepared by mixing with other suitable supplementary ingredients and known additives that can be contained in the food, which are selected by one of ordinary skill in the art. Examples of the foods that can be added include meat, sausage, bread, chocolate, candies, snacks, pizza, ramen, other noodles, gums, dairy products including ice cream, various kinds of soups, beverages, teas, drinks, alcoholic drinks, and vitamin complexes, *etc.* Concentrates, teas, jellies, juices, *etc.* which contain the compound of Formula 1 according to the present invention as the active ingredient can be added in the preparation of the health functional food. Additionally, foods used as feed for animals can also be included.

Still another aspect provides a pharmaceutical composition for preventing or improving wrinkles, which contains a *Symplocarpus foetidus* extract or a fraction thereof as an active ingredient.

As used herein, the terms *"Symplocarpus foetidus",* "extract", "fraction", "wrinkles", "prevention", and "improvement" are as described above.

The pharmaceutical composition of the present invention may be used as a single preparation; may be prepared and used as a combination preparation by further containing a drug that is known to have an effect of improving wrinkles or skin aging; or may be prepared in a unit dosage form by formulating with a pharmaceutically acceptable carrier or excipient or may be prepared by incorporating into a multi-dose container.

The pharmaceutical composition may be administered in a pharmaceutically effective amount.

The term "pharmaceutically effective amount" refers to an amount sufficient to treat diseases at a reasonable benefit/risk ratio applicable to any medical treatment. The effective dose can be determined according to factors, which include the type of a subject and severity, age, sex, drug activity, sensitivity to drug, administration time, administration route and excretion rate, duration of treatment, and other drugs used simultaneously, and other factors well known in the medical field.

To achieve the above objects, still another aspect of the present invention provides a quasi-drug composition for preventing or improving wrinkles, which contains a *Symplocarpus foetidus* extract or a fraction thereof as an active ingredient.

In the present invention, the quasi-drug composition may have the effect of improving skin wrinkles, but the effects of the quasi-drug composition are not limited thereto.

As used herein, the terms *"Symplocarpus foetidus",* "extract", "fraction", "wrinkles", "prevention", and "improvement" are as described above.

The quasi-drug composition of the present invention may further contain a pharmaceutically acceptable carrier, excipient, or diluent as needed, in addition to the above ingredients. The pharmaceutically acceptable carrier, excipient, or diluent is not limited as long as it does not prevent the effects of the present invention, and for example, fillers, extenders, binders, humectants, disintegrants, surfactants, lubricants, sweeteners, fragrances, preservatives, *etc.* may be included.

As used herein, the term "pharmaceutically acceptable carrier" may refer to a carrier, an excipient, or a diluent, which neither stimulates an organism nor inhibits biological activities or properties of a compound to be administered to the organism, and specifically, it may be a non-naturally occurring carrier.

The types of the carrier that can be used in the present invention are not particularly limited, and any carrier can be used as long as it is commonly used in the art and is pharmaceutically acceptable. Non-limiting examples of the carrier may include saline, sterile water, Ringer's solution, buffered saline, an albumin injection solution, a dextrose solution, a maltodextrin solution, glycerol, ethanol, *etc.* The carrier may be used alone or in a combination of two or more kinds thereof.

The composition containing a pharmaceutically acceptable carrier may be in various oral or parenteral formulations. The formulations are prepared using diluents or excipients (*e.g*., fillers, extenders, binders, humectants, disintegrants, surfactants, *etc*.) that are commonly used. Specifically, solid formulations for oral administration may include tablets, pills, powders, granules, capsules, *etc.* These solid formulations may be prepared by mixing the above compound with at least one excipient (*e.g*., starch, calcium carbonate, sucrose, lactose, gelatin, *etc.*)*.* In addition to simple excipients, lubricants (*e.g.,* magnesium stearate and talc) may also be used. Liquid formulations for oral administration may contain suspensions, solutions for internal use, emulsions, syrups, *etc.* In addition to the simple diluents commonly used (*e.g.,* water and liquid paraffin), various other excipients (*e.g*., humectants, sweeteners, fragrances, preservatives, *etc.*) may also be used. Formulations for parenteral administration may contain sterile aqueous solutions, non-aqueous solvents, suspensions, emulsions, lyophilized preparations, and suppositories. As the non-aqueous solvents and the suspensions, propylene glycol, polyethylene glycol, vegetable oil (*e.g.,* olive oil), an injectable ester (*e.g.,* ethyloleate), *etc.* may be used. As a base for the suppositories, Witepsol, Macrogol, Tween 61, cacao butter, laurin butter, glycerogelatin, *etc.* may be used.

The quasi-drug composition of the present invention may be exemplified by disinfectant cleansers, shower foams, ointments, wet tissues, coating agents, *etc.,* but is not limited thereto. The formulation methods, doses, usages, components, *etc.* of quasi-drugs may be appropriately selected from conventional techniques known in the art.

Additionally, the quasi-drug composition of the present invention, which contains a *Symplocarpus foetidus* extract or a fraction thereof as an active ingredient, may be used in a method for improving skin wrinkles, which includes applying the quasi-drug composition to the skin of a subject. The subject includes mammals including mice, cattle, humans, *etc.* without limitation.

Still another aspect of the present invention provides a method for improving wrinkles, which includes administering a composition containing a *Symplocarpus foetidus* extract or a fraction thereof to a subject.

As used herein, the terms *"Symplocarpus foetidus",* "extract", "fraction", and "wrinkles" are as described above.

As used herein, the term "administration" refers to the introduction of the composition of the present invention to a patient by any appropriate manner, and the administration route of the composition may be any conventional route as long as it enables delivery of the composition to the target tissue. The composition may be administered by intraperitoneal, intravenous, intramuscular, subcutaneous, intradermal, oral, topical, or intranasal administration, but the administration route is not limited thereto. As long as the composition of the present invention has the effect of improving skin wrinkles, the composition can be administered by applying it to the skin.

The composition of the present invention may be administered daily or intermittently, and the administration may be administered once or in two or three divided doses per day. Additionally, in order to improve skin wrinkles, the composition of the present invention may be used alone or in combination with another drug treatment. It is important to administer an amount which is capable of obtaining the maximum effect in a minimum amount without side effects in consideration of all of the factors described above, and the amount can easily be determined by one of ordinary skill in the art.

As used herein, the term "subject" refers to all animals including humans, rats, mice, cattle, *etc.* in which wrinkles or skin wrinkles have occurred or can occur, and specifically, the subject may be a mammal including humans.

### [DETAILED DESCRIPTION OF THE INVENTION]

Hereinafter, the present invention will be described in detail with reference to the following Examples. However, these Examples are for illustrative purposes only, and the scope of the invention is not limited by these Examples.

### Example 1: Preparation of Symylocarpus foetidus extract

In order to prepare an extract of *Symplocarpus foetidus* collected from Daedun mountain (Korea), *Symplocarpus foetidus* was pulverized, 10 g of it was added into a flask, and this was extracted by cold-water precipitation for 3 days with 100 g of an extraction solvent (distilled water : methanol = 3 : 7). The extract by cold-water precipitation was filtered through a filter with a pore size of 0.2 µm, and thereby a *Symplocarpus foetidus* extract was prepared.

### Example 2: Measurement of wrinkle-improving activity of Symplocarpus foetidus extract

### 2-1: Experiment on formation of neuromuscular junction and inhibitory effect against muscle contraction

To confirm the inhibitory effect of *Symplocarpus foetidus* against muscle contraction, C2C12 (*i.e.,* a mouse-derived muscle cell line) and NG108-15 *(i.e.,* a mouse-rat hybrid neuronal cell line) were co-cultured.

The muscle cell line, C2C12, was dispensed into a 6-well microplate at a concentration of 5 × 10⁵ cells per well and cultured in a DMEM medium containing 10% bovine serum at 37°C for 24 hours. After removing the medium, the muscle cells were washed once with phosphate buffer, replenished with a DMEM medium containing 2% equine serum, and allowed to differentiate. Two days thereafter, the DMEM medium containing 2% equine serum was replaced with a fresh medium and cultured further for 3 days. Then, the neuronal cell line, NG108-15, was dispensed onto the differentiated muscle cells at a concentration of 5 × 10⁴ cells and cultured in a DMEM medium containing 10% bovine serum at 37°C for 48 hours. Then, the medium of the neuromuscular junction cell model (a co-culture of C2C12 and NG108-15) was removed, and the cells were washed once with phosphate buffer and then treated for 2 hours with a serum-free DMEM medium containing the *Symplocarpus foetidus* extract, which was prepared in Example 1, and with a serum-free DMEM medium not containing a *Symplocarpus foetidus* extract (*i.e.,* a control group), respectively.

The number of muscle contractions was measured using an optical microscope (Zeiss, Oberkochen, Germany) before and after 2 hours of the treatment, and the number of muscle contractions was obtained such that the contraction number was calculated by counting the number of contractions for 30 seconds in three places and averaged.

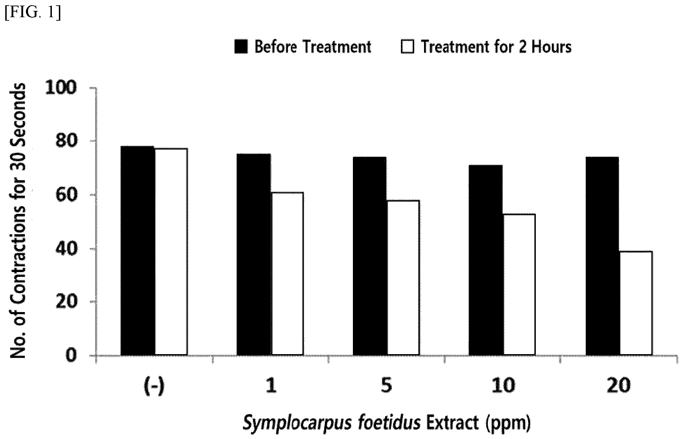

As a result, as shown in FIG. 1 and Table 1, it was confirmed that the *Symplocarpus foetidus* extract reduced muscle contraction in a concentration-dependent manner after 2 hours of treatment, and in particular, it was confirmed that the *Symplocarpus foetidus* extract at a concentration of 20 ppm showed the effect of reducing the number of muscle contractions by about 47%.

**[Table 1]**

| *Symplocarpus foetidus* extract (ppm) | Reduced muscle contraction rate (%) |
|---|---|
| 0 | 1 |
| 1 | 19 |
| 5 | 22 |
| 10 | 25 |
| 20 | 47 |

This means that the *Symplocarpus foetidus* extract can reduce facial expression wrinkles that are being formed by continuous muscle use.

### 2-2: Effect of increasing total amount of collagen biosynthesis

A *Symplocarpus foetidus* extract was added to a culture medium of human-derived fibroblasts so as to test the effect of increasing the total amount of collagen biosynthesis at the cellular level.

Specifically, the measurement of the total amount of collagen increased was quantified using a Procollagen Type I C-Peptide Enzyme Immunoassay Kit (PICP EIA kit). Before the experiment, the cytotoxicity of the *Symplocarpus foetidus* extract prepared in Example 1 was evaluated by varying its concentration (µg/mL) using human-derived fibroblast cells (a method for performing the MTT test by culturing fibroblast cells [reference: Mossman T. (1983). Rapid Colorimetric Assay for Cellular Growth & Survival: application to proliferation & cytotoxicity assays. Journal of Immunological Methods 65, 55-63]), the concentration with no cytotoxicity was selected (40 ppm or less), and the degree of increase in the total amount of collagen was evaluated.

Each sample was added to a culture medium containing human fibroblasts and cultured for 3 days. Then, the culture medium was collected, and the degree of increase in the total amount of collagen at each concentration was measured at 450 nm with a PICP EIA kit using a spectrophotometer. For the comparison of effects, the degree of increase in the total amount of collagen was measured in the samples (*i.e.,* a culture medium (control group) containing fibroblasts where nothing was added and a sample where TGF (positive control group) was added to a concentration of 10 ng/mL) using the same method.

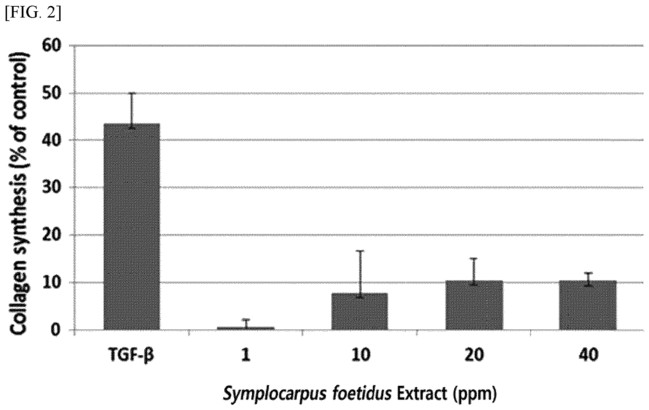

The total amount of collagen was measured as UV absorbance, and the rate of increase of the total amount of collagen was calculated as a ratio of the total amount of collagen relative to that of the control group, and the results are shown in FIG. 2 and Table 2 below.

As a result, as shown in FIG. 2 and Table 2, it was confirmed that the *Symplocarpus foetidus* extract has the effect of increasing the total amount of collagen by promoting collagen synthesis.

**[Table 2]**

| Sample | | Rate of Increase of Collagen Synthesis Relative to that of Control Group (%) |
|---|---|---|
| *Symplocarpus foetidus* | 1 | 0.6 |
| | 5 | 7.8 |
| Extract (ppm) | 10 | 10.4 |
| | 20 | 10.3 |

This means that the *Symplocarpus foetidus* extract can reduce fine wrinkles, the formation of which is known to be caused by a decrease of collagen.

### Example 3: Measurement of effect of cosmetics containing Symplocarpus foetidus on improving wrinkles

Forehead wrinkles are wrinkles which are made by a combination of facial expression wrinkles (formed by continuous use of muscles) and fine wrinkles (formed by aging), and the effect of the *Symplocarpus foetidus* extract of the present invention on the improvement of forehead wrinkles was examined.

Specifically, the essence containing a 2% *Symplocarpus foetidus* extract prepared in Example 1 was applied to 12 healthy women with ages of 30 to 45 on each subject's forehead twice per day for one month.

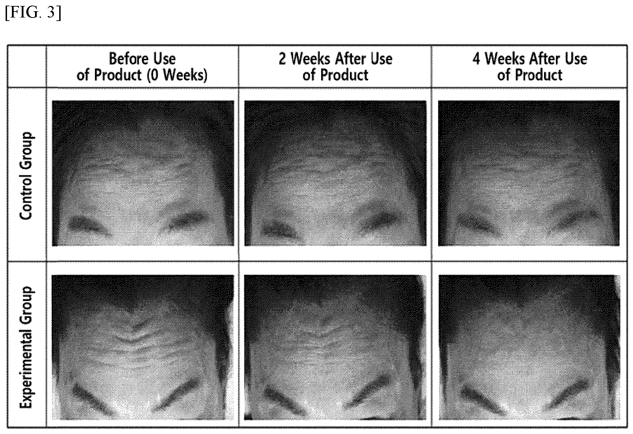

For the control group, an essence that does not contain the *Symplocarpus foetidus* extract was used. The degree of wrinkle improvement was measured through photographic evaluation using a DSLR camera (Nikon DSLR 3100) and questionnaire evaluation, and the results are shown in FIG. 3 and Table 3.

As can be seen in FIG. 3, it was confirmed as a result of the photographic determination that the wrinkles were significantly improved in the experimental group where a 2% *Symplocarpus foetidus* extract of the present invention was used. In addition, with regard to the question on the improvement of skin fine wrinkles and the feeling of skin tightening in the questionnaire evaluation after 4 weeks of use, the average score was 4 points or higher (somewhat agreeable) for the experimental group, whereas the average score was 3 points or higher (somewhat disagreeable) for the control group, thus confirming that fine wrinkles and facial expression wrinkles were improved by the use of the essence containing the 2% *Symplocarpus foetidus* extract (Table 3).

**[Table 3]**

| Questionnaire Items | Experimental Group | | Control Group | |
|---|---|---|---|---|
| | 2 Weeks | 4 Weeks | 2 Weeks | 4 Weeks |
| Fine wrinkles on the forehead skin appear to have been improved. | 4.0 | 4.3 | 2.8 | 3.2 |
| The forehead skin feels tight. | 4.8 | 4.2 | 3.5 | 3.5 |

| | | | | |
|---|---|---|---|---|
| 1 Point: Not agreeable at all. 2 Points: Not agreeable. 3 Points: Somewhat disagreeable. 4 Points: Somewhat agreeable. 5 Points: Agreeable. 6 Points: Certainly agreeable. | | | | |

As a result, can be seen in Table 3, it was confirmed that the use of the essence containing the *Symplocarpus foetidus* extract of Example 1 had the effect of improving forehead wrinkles (facial expression wrinkles and fine wrinkles) compared to the control group which does not contain the *Symplocarpus foetidus* extract.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A cosmetic composition for preventing or improving wrinkles, the composition comprising a *Symplocarpus foetidus* extract or a fraction thereof as an active ingredient.

2. The cosmetic composition of claim 1, wherein the wrinkles are facial expression wrinkles or fine wrinkles.

3. The cosmetic composition of claim 1, wherein the extract is prepared by extracting with a solvent selected from the group consisting of water, a C₁₋₄ alcohol, and a mixed solvent thereof.

4. The cosmetic composition of claim 1, wherein the *Symplocarpus foetidus* extract or fraction thereof is comprised in an amount of 0.0001 wt% to 10 wt% relative to the total weight of the composition.

5. The cosmetic composition of claim 1, wherein the composition reduces facial expression wrinkles by reducing muscle contraction.

6. The cosmetic composition of claim 1, wherein the composition increases the total amount of collagen by promoting collagen synthesis.

7. The cosmetic composition of claim 1, wherein the composition is a formulation selected from the group consisting of a lotion (nutrient emulsion), an astringent lotion, a toner, nourishing cream, massage cream, essence, a pack, a skin adhesive patch, a skin adhesive gel, powder, an ointment, a suspension, an emulsion, a spray, and a cosmetic solution.

8. The cosmetic composition of claim 1, wherein the composition further comprises at least one kind of a carrier selected from the group consisting of oil, water, a surfactant, a moisturizer, a lower alcohol, a thickener, a chelating agent, a pigment, a preservative, and a fragrance.

9. A functional food composition for preventing or improving wrinkles, the functional food composition comprising a *Symplocarpus foetidus* extract or a fraction thereof as an active ingredient.

10. A pharmaceutical composition for preventing or improving wrinkles, the pharmaceutical composition comprising a *Symplocarpus foetidus* extract or a fraction thereof as an active ingredient.

11. A quasi-drug composition for preventing or improving wrinkles, the quasi-drug composition comprising a *Symplocarpus foetidus* extract or a fraction thereof as an active ingredient.

12. A method for improving wrinkles, comprising administering a composition comprising a *Symplocarpus foetidus* extract or a fraction thereof to a subject excluding humans.
